# EUROPÄISCHE PATENTSCHRIFT

(11) **EP 0 792 888 B1**
(45) Veröffentlichungstag und Bekanntmachung des Hinweises auf die Patenterteilung: **21.08.2002**
(21) Anmeldenummer: 97102870.9
(22) Anmeldetag: 21.02.1997
(51) Int. Cl.: C08B 37/18, C11D 1/00, A61K 7/00

(54) **Aliphatische Carbonsäureester von Inulin**
Aliphatic carboxylic acid ester of inulin
Ester d'acide carboxylique aliphatique de l'inuline

(30) Priorität: 01.03.1996 DE 19607847
(43) Veröffentlichungstag der Anmeldung: 03.09.1997
(73) Patentinhaber: SÜDZUCKER AKTIENGESELLSCHAFT MANNHEIM/OCHSENFURT, 68165 Mannheim (DE)
(72) Erfinder: Ehrhardt, Sonja, Dr., 64521 Gross-Gerau (DE); Begli, Alireza Haji, Dr., 67305 Ramsen (DE); Kunz, Markwart, Dr., 67550 Worms (DE); Scheiwe, Linda, 38108 Braunschweig (DE)
(74) Vertreter: Schrell, Andreas, Dr.

(56) Entgegenhaltungen:
- WO-A-96/20266
- CHEMICAL ABSTRACTS, vol. 115, no. 24, 16.Dezember 1991 Columbus, Ohio, US; abstract no. 263084, "Cosmetics containing fructo-oligosaccharide fatty acid esters as emulsifiers or gelling agents" XP002031615 & JP 03 197 409 A (KOBAYASHI KOSE CO LTD ET AL) 28.August 1991
- BULLETIN DES SOCIETES CHIMIQUES BELGES, Bd. 94, Nr. 4, 1985, BE, Seiten 287-291, XP000672623 J. VERMEESCH ET AL: "Synthesis of succinoylated inulin and application as carrier for procainamide"
- MAKROMOLEKULARE CHEMIE. MACROMOLECULAR CHEMISTRY AND PHYSICS, Bd. 187, Nr. 1, 1986, CH, Seiten 125-131, XP002031613 J. VERMEESCH ET AL.: "Synthesis and characterizartion of inulin monosuccinates"
- INULIN AND INULIN-CONTAINING CROPS, 1993, XP002031614 A. FUCHS ED, ELSEVIER SCIENCE PUBLISHERS B.V.:

## Beschreibung

Die Erfindung betrifft aliphatische Carbonsäureester von längerkettigem Inulin, Verfahren zu deren Herstellung und deren Verwendung.

Kohlenhydrate und deren Verbindungen werden seit langem in den verschiedensten Bereichen eingesetzt. Es besteht jedoch nach wie vor Bedarf, neue Verbindungen zu entwickeln, die sich durch verbesserte Eigenschaften, wie beispielsweise erhöhte Grenzflächenaktivität, auszeichnen.

Das lineare, polydisperse Kohlenhydrat Inulin besteht aus β-2-1-verknüpften furanoiden Fructose-Einheiten, deren Kette am reduzierenden Ende von einem α-D-Glucose-Molekül abgeschlossen wird. Es kommt in Compositen wie Zichorien, Topinambur, Dahlien und Artischoken sowie in anderen Pflanzenarten vor, in denen es als Speicherstoff dient. In wirtschaftlich bedeutsamen Mengen wird es aus Zichorien beziehungsweise Topinambur isoliert. Inulin weist in Abhängigkeit von der Pflanzenart und von der Erntezeit verschiedene Molekulargewichtsverteilungen und unterschiedliche durchschnittliche Kettenlängen (DP) auf, die zwischen 8 und 25 liegen. Die Größe der Einzelmoleküle reicht im allgemeinen von 5 bis 50 Monosaccharideinheiten pro Kette. Es gibt aber auch natives Inulin mit DP >25 und entsprechend längeren Einzelketten. Inulin wird technisch vorwiegend auf dem Lebensmittelsektor genutzt, unter anderem zur Herstellung von Diabetikerbrot und Fructosesirup.

Von den bekannten Kohlenhydratestern auf Polysaccharidbasis werden industriell nur die Stärke- und Celluloseester eingesetzt. Stärkeester, insbesondere Acetate, eignen sich im Textilbereich als Schlichtemittel, in der Papierindustrie als Oberflächenleim, in der Film- und Faserindustrie sowie im Nahrungsmittelbereich als Verdickungsmittel und zur Formgebung. Von den Celluloseestern, deren Synthese aufgrund der Schwerlöslichkeit des Ausgangsstoffes sehr aufwendig ist, sind die Acetate und deren Mischester mit Propionat und Butyrat von technischem Interesse. Von den niedermolekularen Kohlenhydratestern finden insbesondere die Saccharoseester Anwendung, insbesondere die Acetate sowie die Mono- und Difettsäureester. Saccharoseacetate beziehungsweise Mischester mit hohem Substitutionsgrad werden als Bleichmittelaktivatoren beziehungsweise Weichmacher verwendet. Saccharosemonofettsäureester werden als Tenside in Reinigungsmitteln und als Emulgatoren eingesetzt.

Die Herstellung von Kohlenhydratestern kann auf verschiedene Weise erfolgen. Im Lösungsmittelverfahren wird das Kohlenhydrat in Gegenwart eines basischen Katalysators in einem Lösungsmittel wie Dimethylformamid oder Dimethylsulfoxid mit Fettsäuremethylestern umgesetzt. Im Mikroemulsionsverfahren dispergiert man den Fettsäureester in einer Lösung des Kohlenhydrates durch einen Emulgator, zum Beispiel durch das entsprechende Alkalisalz der Fettsäure. Das Lösungsmittel wird vor der eigentlichen Reaktion entfernt. Schließlich kann die Umesterung direkt in der Schmelze des Kohlenhydrates mit dem Fettsäureester unter basischer Katalyse stattfinden. Die Reaktionsführung dieser bekannten Verfahren und die Aufarbeitung der Verbindungen ist aufwendig. Die Reaktionsparameter Druck und Temperatur müssen häufig variiert werden. Zur Isolierung des Produktes werden mehrere Extraktions- und Destillationsschritte benötigt. Bei der hohen thermischen Belastung der Produkte besteht zudem die Gefahr der Verfärbung.

Über die Synthese und Verwendung von Inulinestern ist wenig bekannt. JP 63 287 710 beschreibt die Peracetylierung der Inulinkette in Dimethylformamid/Pyridin. Pringsheim et al. (Chem. Ber. 1921, 54, 1281) beschreibt die Peracetylierung der Inulinkette in Pyridin. Das Peracetat zeigt Adhäsionseigenschaften, ist wasserbeständig und wird daher für einen Einsatz im Kosmetikbereich vorgeschlagen. Schacht et al. (Journal of Controlled Release 1985, 2, 245) beschreibt die Verwendung von succinoyliertem Inulin als Trägermaterial für Pharmazeutika.

Die beschriebenen Verbindungen zeichnen sich durch nur geringe Grenzflächenaktivität aus oder sind schwer herzustellen. Tenside, die aus Petrochemikalien hergestellt werden, weisen den Nachteil limitierter Verfügbarkeit auf und sind zudem in ihrer Herstellung nur begrenzt umweltverträglich.

Das der Erfindung zugrundeliegende technische Problem besteht somit in der Bereitstellung von grenzflächenaktiven Kohlenhydratverbindungen, die die vorgenannten Nachteile überwinden, insbesondere einfach herzustellen und umweltverträglich sind sowie aufgrund ihrer grenzflächenaktiven Eigenschaften für eine Vielzahl von Verwendungen in Betracht kommen.

Die Lösung dieses technischen Problems liegt in der Bereitstellung von Inulinestern gemäß Hauptanspruch, Verfahren zu deren Herstellung und deren Verwendung als grenzflächenaktive Substanz. Vorteilhafte Ausführungsformen sind den Unteransprüchen zu entnehmen. Insbesondere betrifft die Erfindung Inulinester, die mindestens 6, bevorzugt 6 bis 50, aber auch mehr als 50 miteinander verbundene Monosaccharideinheiten (Fructoseeinheiten und endständige Glucose) enthalten, wobei von den Hydroxylgruppen des Inulins mindestens eine mit einer gesättigten, 2 bis 22 Kohlenstoffatome aufweisenden Carbonsäure verestert ist, wobei der Substitutionsgrad (DS) < = 0,5 beträgt und wobei die Carbonsäure ausgewählt ist aus der Gruppe bestehend aus Essigsäure, Propionsäure, Buttersäure, Isobuttersäure, Valeriansäure, Capronsäure, Önanthsäure, Caprylsäure, Caprinsäure, Laurinsäure, Myristinsäure, Palmitinsäure, Stearinsäure, Arachinsäure und Behensäure. Im Zusammenhang der vorliegenden Erfindung wird unter einem 6 bis 50 miteinander verbundene Fructoseeinheiten aufweisenden Inulin ein Inulin verstanden, das bevorzugt eine durchschnittliche Kettenlänge von 6 bis 50, aber auch mehr als 50 aufweist, wobei einzelne Ketten durchaus 5 oder mehr als 200 Monosaccharideinheiten enthalten können. Die Erfindung umfaßt selbstverständlich auch Inulinester mit geringfügigen Abweichungen der durchschnittlichen Kettenlänge von dem angegebenen Bereich.

Die erfindungsgemäßen Inulinester sind insbesondere deshalb vorteilhaft, als sich deren Anwendungsmöglichkeiten in hohem Maße durch den Substitutionsgrad (DS) und die Art der Substituenten gezielt steuern lassen. Schon ein geringer Substitutionsgrad bewirkt eine hohe Grenzflächenaktivität. Die erfindungsgemäßen Verbindungen weisen zudem beispielsweise gegenüber Saccharoseestern den Vorteil auf, daß eine größere Anzahl von Hydroxylfunktionen für die Substitution zur Verfügung steht. Dadurch kann bei kaum veränderter Löslichkeit das Verhältnis Hydrophilie/Hydrophobie stärker variiert und damit die Anwendungsmöglichkeiten erweitert werden. Die erfindungsgemäßen Verbindungen weisen gegenüber Stärkeestern den Vorteil auf, daß sie aus einem einheitlichen Rohprodukt hergestellt werden. Zudem sind sie besser löslich als Celluloseester und damit einfacher zu verarbeiten. Schließlich setzen sich die erfindungsgemäßen Inulinester im Gegensatz zu den bekannten Tensiden aus Petrochemikalien nur aus nachwachsenden Rohstoffen zusammen und sind daher biologisch verträglich. Die erfindungsgemäßen Verbindungen oder Gemische davon können beispielsweise aufgrund ihrer hohen Grenzflächenaktivität als Tenside im Wasch-, Spül- und Reinigungsmittelbereich, in der Kosmetik, als Emulgatoren, im Lebensmittel- und Pharmaziebereich, als Additive in der Textil-, Papier- und Lackindustrie verwendet werden.

In einer besonders bevorzugten Ausführungsform betrifft die Erfindung die vorgenannten Inulinester, wobei deren Substitutionsgrad ≤ 0,5 beträgt. Der Substitutionsgrad (DS), der als Mittelwert zu betrachten ist, stellt beim Inulin das Molverhältnis Fructose- (Glucose-) Einheit/Alkylsubstituent dar. Dieser kann maximal 3 betragen, da drei Hydroxylgruppen pro Monosaccharideinheit für die Substitution zur Verfügung stehen. Werte kleiner als 1 bedeuten, daß im Durchschnitt nicht jede Fructose-(Glucose-) Einheit substituiert worden ist. Erfindungsgemäß ist also insbesondere vorgesehen, daß nicht nur Fructoseeinheiten der Inulinkette, sondern auch und nur die endständige Glucoseeinheit substituiert sein kann. Die bevorzugten Inulinester der Erfindung mit einem geringen Substitutionsgrad zeichnen sich in vorteilhafter Weise durch ihre Wasserlöslichkeit aus, wodurch deren Herstellung und Verwendung erheblich vereinfacht und trotzdem eine hohe Oberflächenaktivität erzielt wird. Sie sind daher in hervorragender Weise als Tenside geeignet.

Die Erfindung betrifft auch Inulinester, die durchschnittlich mindestens 6, bevorzugt 6 bis 50, aber auch mehr als 50 miteinander verbundene Fructoseeinheiten enthalten, wobei von den Hydroxylgruppen des Inulins mindestens eine mit einer gesättigten 2 bis 7 Kohlenstoffatome aufweisenden Carbonsäure verestert ist. Die Erfindung betrifft demgemäß in einer besonders bevorzugten Ausführungsform eine Inulinkette, die mit kurzkettigen verzweigten oder unverzweigten Carbonsäuren verestert ist. Die erfindungsgemäß bevorzugten Carbonsäuren sind Essigsäure, Propionsäure, Buttersäure, Isobuttersäure, Valeriansäure, Capronsäure oder Önanthsäure. Die Vorteile dieser Inulinester mit kurzkettigen Carbonsäureresten und niedrigeren bis mittleren DS liegen in ihrer hohen Wirksamkeit bei der Verringerung der Oberflächenspannung von Wasser. Ferner bilden diese Ester schon bei geringer Konzentration Micellen, wodurch deren besondere Eignung als Additiv in Reinigungsmitteln begründet wird. Derartige Inulinester weisen beispielsweise in Folien geringe Migarationsneigung auf und sind daher ausgezeichnet als Weichmacher zu verwenden.

Die Erfindung betrifft auch die vorgenannten Inulinester, wobei die Hydroxylgruppen mit identischen oder unterschiedlichen Carbonsäuren verestert sind.

Die Erfindung betrifft ferner wässrige Lösungen und Pulver, die einen Inulinester der Erfindung enthalten.

In einer besonders bevorzugten Ausführungsform betrifft die Erfindung manuelle Geschirrspülmittel, Reinigungscremes, Haarspülmittel und Körperlotions, die die erfindungsgemäßen Inulinester, beispielsweise Inulinlaurat (zum Beispiel DS 0,04), Inulinpalmitat (zum Beispiel DS 0,04) oder Inulinstearat (zum Beispiel DS 0,04) enthalten.

In einer weiteren Ausführungform der Erfindung wird ein Verfahren zur Herstellung eines der vorgenannten Inulinesters bereitgestellt, wobei mindestens 6, bevorzugt 6 bis 50, aber auch mehr als 50 miteinander verbundene Fructoseeinheiten aufweisendes Inulin mit Chloriden oder Anhydriden 2 bis 22, vorzugsweise 2 bis 7, Kohlenstoffatome aufweisender Carbonsäuren oder Gemischen davon umgesetzt wird, wobei das einzige Lösungsmittel Pyridin ist.

Die Erfindung sieht ferner ein Verfahren zur Herstellung eines erfindungsgemäßen Inulinesters vor, wobei mindestens 6, bevorzugt 6 bis 50, aber auch mehr als 50 miteinander verbundene Fructoseeinheiten aufweisendes Inulin mit Anhydriden 2 bis 22, vorzugsweise 2 bis 7, Kohlenstoffatome aufweisender Carbonsäuren oder Gemischen davon umgesetzt wird, wobei als einziges Losungsmittel Wasser verwendet wird.

Schließlich sieht die Erfindung ein Verfahren zur Herstellung eines erfindungsgemäßen Inulinesters vor, wobei mindestens 6, bevorzugt 6 bis 50, aber auch mehr als 50 Fructoseeinheiten aufweisendes Inulin mit Anhydriden 2 bis 22, vorzugsweise 2 bis 7, Kohlenstoffatome aufweisender Carbonsäuren oder Gemischen davon lösungsmittelfrei umgesetzt wird.

Die Erfindung erfaßt auch ein Verfahren zur Herstellung eines erfindungsgemäßen Inulinesters, wobei mindestens 6, bevorzugt 6 bis 50, aber auch mehr als 50 miteinander verbundene Monosaccharideinheiten aufweisendes Inulin mit Methyl- oder Ethylestern, 2 bis 22 Kohlenstoffatome aufweisender Carbonsäuren lösungsmittelfrei vorzugsweise in Knetern, besonders bevorzugt in Extrudern umgesetzt wird. Die Umsetzung läßt sich insbesondere durch Verwendung von Estern 12 bis 22 Kohlenstoffatome aufweisender Carbonsäuren verbessern. Durch den Kneter oder besonders durch den Extruder laßt sich die thermische Belastung reduzieren, und es werden die erwähnten Schwierigkeiten des Schmelzverfahrens vermieden.

Die erfindungsgemäßen Verbindungen werden also durch Umsetzung von Inulin mit einer mittleren, durchschnittlichen Kettenlänge von 6, bevorzugt zwischen 6 und 50, aber auch mehr als 50, mit Carbonsäurechloriden oder Anhydriden, C2 bis C22, bevorzugt C2. bis C7, mit oder ohne Lösungsmittel, mit ' oder ohne Katalysator hergestellt. Bei der Verwendung von Carbonsäuremethyl- oder -ethylestern (C2 bis C22, vorzugsweise C12 bis C22) werden diese mit Inulin mit einer mittleren durchschnittlichen Kettenlänge von 6, bevorzugt zwischen 6 bis 50, aber auch mehr als 50, lösungsmittelfrei mit Katalysator umgesetzt.

Die erfindungsgemäß hergestellten Inulinester werden als farblose Produkte erhalten, deren Eigenschaftsprofil vom Substituenten und vom Substitutionsgrad geprägt wird. Über das Molverhältnis der Edukte lassen sich Substitutionsgrade im Bereich von 0,03 bis ≤0,5 einstellen.

Die Erfindung sieht insbesondere vor, daß Inulin in Pyridin, in Wasser oder lösungsmittelfrei mit Carbonsäureanhydrid in Gegenwart eines Katalysators oder mit Carbonsäurechloriden in Pyridin mit oder ohne Katalysator verestert wird, vorzugsweise in einem Mengenverhältnis von Inulin zu Anhydrid oder Inulin zu Chlorid von 36:1 bis 1:8.

Der Erfindung gemäß werden insbesondere Anhydride oder Säurechloride der folgenden Carbonsäuren oder physikalische Gemische derselben verwendet:

Essigsäure, Propionsäure, Isobuttersäure, Buttersäure, Valeriansäure, Capronsäure, Önanthsäure, Caprylsäure, Caprinsäure, Laurinsäure, Myristinsäure, Palmitinsäure, Stearinsäure, Arachinsäure oder Behensäure.

Im erfindungsgemäßen Knet- oder Extrudierverfahren wird Inulin lösungsmittelfrei unter Verwendung eines Katalysators mit Carbonsäuremethyl- oder -ethylester (C2 bis C22, vorzugsweise C12 bis C22) verestert, vorzugsweise in einem Mengenverhältnis von Inulin zu Ester von 36:1 bis 1:8.

Der Erfindung gemäß werden Carbonsäureester, insbesondere Methyl- oder Ethylester der folgenden Carbonsäuren verwendet:

Laurin-, Myristin-, Palmitin-, Stearin-, Arachinoder Behensäure.

Die erfindungsgemäßen Verfahren können in Gegenwart eines Katalysators durchgeführt werden. Erfindungsgemäß kommen insbesondere basische oder saure Katalysatoren in Betracht, wie 4-(Dimethylamino)-pyridin, Natriumacetat, Kaliumcarbonat, Ionenaustauscher in der H⁺-Form, OH⁻-Form oder Pyridin sowie weitere basische Verbindungen.

Die Veresterung erfolgt lösungsmittelfrei bei Temperaturen von 100° C bis 150° C und bei Verwendung von Lösungsmitteln bei Temperaturen von 20° C bis 100° C, vorzugsweise 25° C bis 60° C.

Die Produkte werden IR- und NMR-spektroskopisch identifiziert, wobei folgende Werte charakteristisch sind:

### IR-Signale:

Die Valenzschwingung der Alkylsubstituenten liegt bei 2940 cm⁻¹, die Carbonylvalenzschwingung der Ester bei 1750 cm⁻¹ und die Kohlenstoff-Sauerstoff-Valenzschwingungen zwischen 1100 cm⁻¹ und 1300 cm⁻¹.

### ¹H-NMR-Signale:

Die Resonanzsignale der Alkylsubstituenten liegen bei 2,3 ppm und zwischen 0,8 ppm und 1,6 ppm, während die Signale der Inulinkette zwischen 3,4 ppm und 5,7 ppm liegen.

### ¹³C-NMR-Signale:

Die Carbonylkohlenstoffsignale liegen im Bereich von 172 bis 180 ppm, die Signale der Alkylsubstituenten von 15 bis 30 ppm und die Signale der Inulinkette bei 102 bis 105 ppm, 74 bis 82 ppm und 60 bis 63 ppm.

Der Substitutionsgrad wird aus dem Protonenverhältnis Substituent/Inulin ermittelt, das durch die Integration des ¹H-NMR-Signals erhalten wird.

Die folgenden Beispiele erläutern die Erfindung:

### Beispiel 1:

### Veresterung von Inulin mit Acetanhydrid in Pyridin

In einem temperierbaren Reaktor mit Rührwerk werden 21 g (0,13 Mol) Inulin, gelöst in 140 mL Pyridin, mit 1,2 mL (0,013 Mol) Acetanhydrid versetzt. Das Produkt wird nach 24 h bei 25° C aus der Reaktionsmischung mit 100 mL Essigsäureethylester gefallt, abgesaugt und in 100 mL Wasser aufgenommen. Nach Erhalt einer Suspension wird diese zentrifugiert und die überstehende klare Losung lyophilisiert. Das Produkt wird in 85 Gew.-% Ausbeute erhalten. Der Substitutionsgrad beträgt 0,03 und die Oberflächenspannung einer 1 Gew.-% Lösung 66,43 mN/m.

Analog lassen sich Inulinacetate mit anderen Substitutionsgraden durch Variation der Acetanhydridmenge herstellen.

### Beispiel 2:

### Veresterung von Inulin mit Buttersäureanhydrid in Pyridin

In einem temperierbaren Reaktor mit Rührwerk werden 21 g (0,13 Mol) Inulin, gelöst in 140 mL Pyridin, mit 2,1 mL (0,013 Mol) Buttersäureanhydrid versetzt. Das Produkt wird nach 24 h bei 25° C aus der Reaktionsmischung mit 100 mL Essigsäureethylester gefällt, abgesaugt und in 100 mL Wasser aufgenommen. Nach Erhalt einer Suspension wird diese zentrifugiert und die überstehende klare Lösung lyophilisiert. Das Produkt wird in 85 Gew.-% Ausbeute erhalten. Der Substitutionsgrad betragt 0,06 und die Oberflächenspannung einer 1 Gew.-% Lösung 54,95 mN/m.

### Beispiel 3:

### Veresterung von Inulin mit Capronsäureanhydrid in Pyridin

In einem temperierbaren Reaktor mit Rührwerk werden 21 g (0,13 Mol) Inulin, gelöst in 140 mL Pyridin, mit 2,6 mL (0,013 Mol) Capronsäureanhydrid versetzt. Das Produkt wird nach 24 h bei 25° C aus der Reaktionsmischung mit 100 mL Essigsäureethylester gefällt, abgesaugt, in 100 mL Wasser aufgenommen. Nach Erhalt einer Suspension wird diese zentrifugiert und die überstehende klare Lösung lyophilisiert. Das Produkt wird in 75 Gew.-% Ausbeute erhalten. Der Substitutionsgrad beträgt 0,04 und die Oberflächenspannung einer 1 Gew.-% Lösung 43,73 mN/m.

### Beispiel 4:

### Veresterung von Inulin mit Caprylsäureanhydrid in Pyridin

In einem temperierbaren Reaktor mit Rührwerk werden 21 g (0,13 Mol) Inulin, gelöst in 150 mL Pyridin, mit 3,5 g (0,013 Mol) Caprylsäureanhydrid versetzt. Das Produkt wird nach 24 h bis 25° C aus der Reaktionsmischung mit 100 mL Essigsäureethylester gefällt, abgesaugt und in 100 mL Wasser aufgenommen. Nach Erhalt einer Suspension wird diese zentrifugiert und die überstehende klare Lösung lyophilisiert. Das Produkt wird in 75 Gew.-% Ausbeute erhalten. Der Substitutionsgrad beträgt 0,04 und die Oberflachenspannung einer 1 Gew.-% Lösung 37,33 mN/m.

### Beispiel 5:

### Veresterung von Inulin mit Caprinsäureanhydrid in Pyridin

In einem temperierbaren Reaktor mit Rührwerk werden 21 g (0,13 Mol) Inulin, gelöst in 150 mL Pyridin, mit 4,2 g (0,013 Mol) Caprinsäureanhydrid versetzt. Das Produkt wird nach 24 h bei 25° C aus der Reaktionsmischung mit 100 mL Essigsäureethylester gefällt, abgesaugt und in 100 mL Wasser aufgenommen. Nach Erhalt einer Suspension wird diese zentrifugiert und die überstehende klare Lösung lyophilisiert. Das Produkt wird in 85 Gew.-% Ausbeute erhalten. Der Substitutionsgrad beträgt 0,04 und die Oberflächenspannung einer 1 Gew.-% Lösung 34,23 mN/m.

### Beispiel 6:

### Veresterung von Inulin mit Laurinsäureanhydrid in Pyridin

In einem temperierbaren Reaktor mit Rührwerk werden 21 g (0,13 Mol) Inulin, gelöst in 150 mL Pyridin, mit 4,9 g (0,013 Mol) Laurinsäureanhydrid versetzt. Das Produkt wird nach 24 h bei 25° C aus der Reaktionsmischung mit 100 mL Essigsäureethylester gefällt, abgesaugt und in 100 mL Wasser aufgenommen. Nach Erhalt einer Suspension wird diese zentrifugiert und die überstehende klare Lösung lyophilisiert. Das Produkt wird in 80 Gew.-% Ausbeute erhalten. Der Substitutionsgrad beträgt 0,04 und die Oberflächenspannung einer 1 Gew.-% Lösung 38,17 mN/m.

### Beispiel 7:

### Veresterung von Inulin mit Myristinsäureanhydrid in Pyridin

In einem temperierbaren Reaktor mit Rührwerk werden 21 g (0,13 Mol) Inulin, gelöst in 150 mL Pyridin, mit 5,67 g (0,013 Mol) Myristinsäureanhydrid versetzt. Das Produkt wird nach 24 h bei 25° C aus der Reaktionsmischung mit 100 mL Essigsäureethylester gefällt, abgesaugt und in 100 mL Wasser aufgenommen. Nach Erhalt einer Suspension wird diese zentrifugiert und die überstehende klare Lösung lyophilisiert. Das Produkt wird in 80 Gew.-% Ausbeute erhalten. Der Substitutionsgrad beträgt 0,04 und die Oberflächenspannung einer 1 Gew.-% Lösung 34,29 mN/m.

### Beispiel 8:

### Veresterung von Inulin mit Palmitinsaureanhydrid in Pyridin

In einem temperierbaren Reaktor mit Rührwerk werden 21 g (0,13 Mol) Inulin, gelöst in 150 mL Pyridin, mit 6,4 g (0,013 Mol) Palmitinsäureanhydrid versetzt. Das Produkt wird nach 24 h bei 25° C aus der Reaktionsmischung mit 100 mL Essigsäureethylester gefällt, abgesaugt und in 100 mL Wasser aufgenommen. Nach Erhalt einer Suspension wird diese zentrifugiert und die überstehende klare Lösung lyophilisiert. Das Produkt wird in 80 Gew.-% Ausbeute erhalten. Der Substitutionsgrad beträgt 0,04 und die Oberflächenspannung einer 1 Gew.-% Lösung 30,15 mN/m.

### Beispiel 9:

### Veresterung von Inulin mit Caprinsäurechlorid in Pyridin

In einem temperierbaren Reaktor mit Rührwerk werden 21 g (0,13 Mol) Inulin, gelöst in 150 mL Pyridin, mit 83,0 mL (0,4 Mol) Caprinsäurechlorid versetzt. Nach 24 h bei 40° C wird mit 100 mL Wasser hydrolysiert und das Produkt mit Essigsäureethylester (3 x 100 mL) extrahiert. Nach Waschen der Essigester-Phase mit 1 M Natronlauge (3 x 30 mL) und Abdestillieren des Essigesters unter Vakuum wird das Produkt als braunes Öl erhalten. Die Entfärbung erfolgt mit Aktivkohle (Ausbeute 65 Gew.-%, DS 2,5).

### Beispiel 10:

### Veresterung von Inulin mit Stearinsäurechlorid in Pyridin mit 4-(Dimethylamino)-pyridin als Katalysator

In einem temperierbaren Reaktor mit Rührwerk werden 15 g (0,09 Mol) Inulin, gelöst in 250 mL Pyridin, mit 1,3 g (0,01 Mol) 4-Dimethylamino-pyridin und mit 60 mL (0,18 Mol) Stearinsäurechlorid versetzt. Nach 24 h bei 60° C wird mit 100 mL Wasser hydrolysiert und das Produkt mit Essigsäureethylester (4 x 100 mL) extrahiert. Nach Waschen der Essigester-Phase mit 1 M Natronlauge (3 x 30 mL), mit 20 mL 50 Gew.-% Essigsäure und Abdestillieren des Essigesters unter Vakuum wird das Produkt als farbloser Feststoff in 62 Gew.-% Ausbeute erhalten, DS 1,8.

### Beispiel 11:

### Veresterung von Inulin mit Stearinsäureanhydrid in Pyridin mit 4-(Dimethylamino)-pyridin als Katalysator

In einem temperierbaren Reaktor mit Rührwerk werden 10 g (0,06 Mol) Inulin, gelöst in 100 mL Pyridin, mit 0,6 g (0,005 Mol) 4-Dimethylamino-pyridin und mit 98 g (0,18 Mol) Stearinsäureanhydrid versetzt. Nach 24 h bei 60° C wird mit 100 mL Wasser hydrolysiert, filtriert und das Filtrat mit Essigsäureethylester (4 x 100 mL) extrahiert. Nach Waschen der Essigester-Phase mit 20 mL 50 Gew.-% Essigsäure und Abdestillieren des Essigesters unter Vakuum wird das Produkt als farbloser Feststoff in 45 Gew.-% Ausbeute erhalten, DS 2,3.

### Beispiel 12:

### Veresterung von Inulin lösungsmittelfrei mit Isobuttersäureanhydrid in Gegenwart von Natriumacetat

In einem temperierbaren Reaktor mit Ruhrwerk wird eine Mischung aus 15 g (0,09 Mol) Inulin und 0,75 g (0,009 Mol) Natriumacetat, suspendiert in 66,5 mL (0,4 Mol) Isobuttersäureanhydrid, 4 h bei 140° C erhitzt. Im Vakuum (15 hPa) wird das überschüssige Anhydrid entfernt. Der Rückstand wird in 100 mL Essigester aufgenommen, mit Wasser (3 x 30 mL), 1 M Natronlauge (3 x 30 mL) und erneut mit Wasser (3 x 30 mL) gewaschen. Nach Entfernung des Lösungsmittels wird das Produkt als leicht gefärbtes ol erhalten (Ausbeute 75 Gew.-%, DS 3).

### Beispiel 13:

### Veresterung von Inulin lösungsmittelfrei mit Essigsäureanhydrid/Isobuttersaureanhydrid in Gegenwart von Natriumacetat

In einem temperierbaren Reaktor mit Rührwerk werden 10 g (0,06 Mol) Inulin in einer Lösung aus 10 mL (0,09 Mol) Essigsäureanhydrid und 15 mL (0,09 Mol) Isobuttersäureanhydrid suspendiert, mit 0,5 g (0,006 Mol) Natriumacetat versetzt und 4 h bei 140° C erhitzt. Im Vakuum (15 hPa) wird das überschüssige Anhydrid entfernt. Der Rückstand wird in 100 mL Essigester aufgenommen, mit Wasser (3 x 30 mL), 1 M Natronlauge (3 x 30 mL) und erneut mit Wasser (3 x 30 mL) gewaschen. Nach Entfernung des Lösungsmittels wird das Produkt als leicht gefärbtes Öl erhalten (Ausbeute 60 Gew.-%, DS 1,5).

### Beispiel 14:

### Veresterung von Inulin in Wasser mit Propionsäureanhydrid in Gegenwart eines Ionenaustauschers

In einem temperierbaren Reaktor mit Rührwerk werden 15 g (0,09 Mol) Inulin, gelöst in 250 mL Wasser, mit 12,3 mL (0,09 Mol) Propionsäureanhydrid versetzt und in Gegenwart von 300 g Merck Ionenaustauscher III (OH⁻-Form) 24 h bei 40° C erwärmt. Der Ionenaustauscher wird abfiltriert, die Lösung wird im Vakuum eingeengt, der Rückstand in 100 mL Wasser suspendiert und das Gemisch filtriert. Aus dem Filtrat wird durch Gefriertrocknung das Produkt als farbloser Feststoff in 50 Gew.-% Ausbeute erhalten, DS = 0,5.

### Beispiel 15:

### Veresterung von Inulin lösungsmittelfrei mit Essigsäureanhydrid in Gegenwart eines Ionenaustauschers

In einem temperierbaren Reaktor mit Rührwerk werden 10 g (0,06 Mol) Inulin in 13,5 mL (0,12 Mol) Essigsäureanhydrid suspendiert und in Gegenwart von 0,3 g Amberlite IR 120 (H⁺-Form) 0,5 h bei 100° C erwärmt. Der Ionenaustauscher wird abfiltriert, die Lösung im Vakuum eingeengt und der Rückstand in 100 mL Wasser suspendiert. Aus dem Filtrat der Suspension wird durch Gefriertrocknung das Produkt als farbloser Feststoff in 30 Gew.-% Ausbeute erhalten, DS 0,8.

### Beispiel 16:

### Veresterung von Inulin mit Caprinsäurechlorid in Pyridin

In einem temperierbaren Reaktor mit Ruhrwerk werden 21 g (0,13 Mol) Inulin, gelöst in 140 mL Pyridin, mit 13,5 mL (0,065 Mol) Caprinsäurechlorid versetzt. Nach 24 h bei 40° C wird die Reaktionsmischung mit 100 mL Wasser hydrolysiert und mit Essigsäureethylester (4 x 100 mL) extrahiert. Nach Waschen der Essigester-Phase mit 1 M Natronlauge (3 x 30 mL) und Abdestillieren des Essigesters unter Vakuum wird das Produkt mit einem Substitutionsgrad von 0,4 in 60%-Ausbeute erhalten. Die Oberflächenspannung einer 0,03 Gew.-%-Lösung beträgt 40 mN/m.

### Beispiel 17:

### Veresterung von Inulin mit Stearinsäurechlorid in Pyridin mit 4-(Dimethylamino)-pyridin als Katalysator

In einem temperierbaren Reaktor mit Rührwerk werden 15 g (0,09 Mol) Inulin, gelöst in 150 mL Pyridin, mit 0,7 g (0,005 Mol) 4-(Dimethylamino)-pyridin und mit 30 mL (0,09 Mol) Stearinsäurechlorid versetzt. Nach 24 h bei 60° C wird die Reaktionsmischung mit 100 mL Wasser hydrolysiert und mit Essigsäureethylester (4 x 100 mL) extrahiert. Nach Waschen der Essigester-Phase mit 1 M Natronlauge (3 x 30 mL) und Abdestillieren des Essigesters unter Vakuum wird das Produkt mit einem Substitutionsgrad von 0,8 in 65 %-Ausbeute erhalten. Die Oberflächenspannung einer 0,03 Gew.-%-Losung beträgt 28 mN/m.

### Beispiel 18:

### Veresterung von Inulin mit Stearinsäureanhydrid in Pyridin mit 4-(Dimethylamino)-pryridin als Katalysator

In einem temperierten Reaktor mit Rührwerk werden 21 g (0,13 Mol) Inulin, gelöst in 150 mL Pyridin, mit 1,3 g (0,01 Mol) 4-(Dimethylamino)-pyridin und mit 35 g (0,065 Mol) Stearinsaureanhydrid, gelöst in 150 mL Pyridin, versetzt. Nach 24 h bei 60° C wird die Reaktionsmischung mit 200 mL Wasser hydrolysiert und mit Essigsäureethylester (5 x 100 mL) extrahiert. Nach Waschen der Essigester-Phase mit 1 M Natronlauge (3 x 30 mL) und Abdestillieren des Essigesters unter Vakuum wird das Produkt mit einem Substitutionsgrad von 0,4 in 55 %-Ausbeute erhalten. Die Oberflächenspannung einer 0,03 Gew.-%-Lösung beträgt 35 mN/m.

### Beispiel 19:

### Lösungsmittelfreie Acylierung von Inulin mit Palmitinsäuremethylester

Über einen Doppelschneckenextruder wird eine Mischung aus 10 kg (60 Mol) Inulin, 11,6 kg (43 Mol) Palmitinsäuremethylester, 1,62 kg (4 Mol) Kaliumstearat und 0,30 kg (2 Mol) Kaliumcarbonat in einen Reaktor extrudiert und 60 min bei 100 mbar auf 150°C erwärmt. Das erhaltene Rohprodukt wird in 200 L Wasser suspendiert, filtriert, das Filtrat mit Diethylether (4 x 50 L) extrahiert und gefriergetrocknet. In einer Soxhlet-Apparatur wird das Lyophilisat mit siedendem Butanol (400 L) 16 h extrahiert. Aus der eingeengten Extraktphase läßt sich das Produkt als leicht gefärbter Feststoff in 50 Gew.-% Ausbeute erhalten. DS = 0,05, σ = 30,0 mN/m (1% Lösung).

### Beispiel 20:

### Bestimmung der Oberflächenspannung

Die erfindungsgemäßen Produkte weisen eine hohe Oberflächenaktivität schon bei minimalen Substitutionsgraden auf. Die Oberflächenspannung σ wässriger Inulinesterlösungen (1 Gew.-%) wurde bei 25°C nach der Wilhelmy-Methode gemessen. Die in der nachstehenden Tabelle 1 aufgeführte Probenbezeichnung wird durch den in Klammern angegebenen Substitutionsgrad ergänzt.

**Tabelle 1:**

| Bestimmung der Oberflachenspannung | |
|---|---|
| **Probe** | **σ (mN/m)** |
| Inulinacetat (0,03) | 66,43 |
| Inulinacetat (0,7) | 51,14 |
| Inulinbutyrat (0,06) | 54,95 |
| Inulincapronat (0,04) | 43,73 |
| Inulincaprylat (0,04) | 37,33 |
| Inulincaprinat (0,04) | 34,23 |
| Inulinlaurat (0,04) | 38,17 |
| Inulinmyristat (0,05) | 34,29 |
| Inulinpalmitat (0,05) | 30,15 |

### Beispiel 21:

### Solubilisierung von Sudan Rot B in Wasser mit Inulinestern

Inulinesterlösungen verschiedener Konzentrationen (0,06 g, 0,12 g und 0,24 g in 20 mL Wasser) wurden jeweils mit 0,01 g Sudan Rot B versetzt. Der Farbstoff wurde durch Ultraschall dispergiert und anschließend die erhaltene Suspension 60 min bei 7000 U/min zentrifugiert. Die Extinktion der uberstehenden klaren Lösung wurde photometrisch bei einer Wellenlänge von 516 nm (Küvettenlange 1 cm) gemessen. Als Nullprobe diente eine Losung von Sudan Rot B in Wasser (0,01 g in 20 mL).

In Tabelle 2 sind beispielhaft die Ergebnisse von Inulinstearat (DS 0,04) angegeben. Es zeigt sich, daß Inulinester den Farbstoff solubilisiert. Diese Solubilisierungseigenschaften verdeutlichen die erfindungsgemäß vorgesehene Verwendung von Inulinestern in Wasch-, Reinigungs- und Färbemitteln.

**Tabelle 2:**

| Solubilisierung | |
|---|---|
| **Inulinstcaratkonzentration** **(Gew.-%; DS 0,04)** | **Extinktion** |
| 0 | 0,113 |
| 0,3 | 0,263 |
| 0,6 | 0,400 |
| 1,2 | 0,407 |

### Beispiel 22:

### Benetzungseigenschaften von Inulinestern

Die Benetzung von Festkörperoberflächen durch wäßrige Lösungen von Inulinestern wurde exemplarisch an Glas mit einer 1 Gew.-% wäßrigen Losung von Inulinestern untersucht. Die Messung erfolgte mit der Wilhelmymethode, als Meßgröße dient der Randwinkel, der als Fortschreitwinkel angegeben wird (s.Tabelle 3). Die erfindungsgemäßen Inulinester erhohen die Benetzung von Glas im Vergleich zu reinem Wasser.

**Tabelle 3:**

| Benetzung von Glas | |
|---|---|
| **Substanz** | **Fortschreitwinkel [°]** |
| Wasser | 45 |
| 1 Gew.-% Inulincapronatlösung (DS 0,04) | 38 |

### Beispiel 23:

### Beschichtungseigenschaften von Inulinestern

Werden Festkörper längere Zeit in eine wäßrige Lösung von Inulinestern getaucht, verändert sich die Benetzung des Festkörpers durch Wasser, wie die folgenden Meßergebnisse verdeutlichen. Als Probekörper wurde Teflon verwandt, als Inulinesterlösung eine 1 Gew.-% Inulinpalmitatlösung (DS 0,04); die Eintauchzeit betrug 30 min. Der Fortschreitwinkel wurde vermindert von 88° auf 73°.

### Beispiel 24:

### Viskosität wäßriger Inulinesterlösungen

Durch die Zugabe von Inulinester wird die Viskosität von Wasser erhöht, wie die Viskositätsmessungen wäßriger Inulinesterlosungen mit dem Ubbelohdeviskosimeter belegen (siehe Tabelle 4). Die Temperatur betrug 25° C.

**Tabelle 4:**

| Viskositätsmessungen | | |
|---|---|---|
| Substanz | Konzentration (10⁻¹g/mL) | Viskosität (mm²/s) |
| Wasser | -- | 0,942 |
| Inulin | 1 | 1,46 |
| Inulincapronat (DS 0,05) | 1 | 1,72 |
| Inulincapronat (DS 0,1) | 1 | 1,79 |
| Inulinstearat (DS 0,04) | 1 | 3,01 |

### Beispiel 25:

### Weichmachereigenschaften von Inulinestern

Werden Folien aus Celluloseacetat in Gegenwart von Inulinestern mit hohem Substitutionsgrad (DS >1) und kurzkettigen Alkylsubstituenten hergestellt, so wird die Härte der Folien vermindert und die Flexibilität erhöht, ohne daß sich die Transparenz der Folien verschlechtert. Die Tabelle 5 erfaßt die Ergebnisse einiger beispielhafter Untersuchungen. Die Härte der Folien wurde durch mehrere Personen im Fingertest und die Flexibilität im Biegetest ermittelt.

**Tabelle 5:**

| Weichmachertest mit Celluloseacetat (1,2 g) und 0,8 g Weichmacher (LS-Inulin : Inulin DP 5-6, DS des Celluloseacetat: 2) | | | | |
|---|---|---|---|---|
| Folie | Farbe, optische Eigenschaft | Durchläßigkeit: IR[%] | Härte | Flexibilität |
| Celluloseacetat | farblos, klar | 35 | sehr hoch | sehr gering |
| Celluloseacetat - Inulin-acetatisobutyrat DS 1,4, Acetat DS=0,4 | farblos, klar | 25 | gering | hoch |
| Celluloseacetat+Inulinacetatisobutyrat DS 2, Acetat DS 1,7 | farblos, klar | 23 | gering | hoch |
| Celluloseacetat + LS-Inulinacetatisobutyrat DS 1,4 | farblos, klar | 22 | gering | hoch |
| Celluloseacetat + Inulin-acetatpropionat DS 1,5 | farblos, klar | 20 | gering | hoch |
| Celluloseacetat + Inulinisobutyratpropionat DS 1,4 | farblos, klar | 20 | gering | hoch |
| Celluloseacetat + Inulin-capronat DS-3 | farblos, klar | 18 | gering | hoch |

### Beispiel 26:

### Rezeptur für ein manuelles Geschirrspülmittel:

Zur Herstellung eines erfindungsgemäßen manuellen Geschirrspülmittels werden 30 g Inulinlaurat (DS 0,04) (30 Gew.-%) mit 6 ml Ethanol (6 Gew.-%), 1 g Natriumchlorid (1 Gew.-%), 0,5 g Konservierungsmittel (0,5 Gew.-%) und 0,5 g Parfumol (0,5 Gew.-%) gemischt und mit 62 ml Wasser (62 Gew.-%) auf 100 g aufgefüllt.

### Beispiel 27:

### Herstellung einer Reinigungscreme:

Zur Herstellung einer erfindungsgemäßen Reinigungscreme werden 8 g (8 Gew.-%) Inulinlaurat (DS 0,04) mit 4 g Cetanol (4 Gew.-%), 3 g Stearinsäure (3 Gew.-%), 15 g Paraffinöl (15 Gew.-%), 0,2 g Konservierungsmittel (0,2 Gew.-%) und 0,3 g Parfümöl (0,3 Gew.-%) gemischt und mit 69,5 g Wasser (69,5 Gew.-%) auf 100 g aufgefüllt.

### Beispiel 28:

### Herstellung eines Haarspülmittels:

Zur Herstellung eines erfindungsgemäßen Haarspülmittels werden 6 g (6 Gew.-%) Inulinstearat (DS 0,04) mit 6 g (6 Gew.-%) Lauryltrimethylammoniumchlorid, 4 g (4 Gew.-%) Stearylalkohol, 4 g (4 Gew.-%) Glycerin, 1 g (1 Gew.-%) Citronensäure und 0,5 g (0,5 Gew.-%) Parfumol gemischt und mit 78,5 g (78,5 Gew.-%) Wasser auf 100 g aufgefüllt.

### Beispiel 29:

### Herstellung einer Körperlotion:

Zur Herstellung einer erfindungsgemäßen Korperlotion werden 8 g (8 Gew.-%) Inulinpalmitat (DS 0,04) mit 2 g (2 Gew.-%) Cetanol, 2 g (2 Gew.-%) Isopropylmyristat, 4 g (4 Gew.-%) Polysiloxan, 4 g (4 Gew.-%) Palmitinsäuretriglycerid, 4 g (4 Gew.-%) 1,2-Propandiol, 3 g (3 Gew.-%) Carbomer und 0,5 g (0,5 Gew.-%) Parfümöl gemischt und mit 72,5 g (72,5 Gew.-%) Wasser auf 100 g aufgefüllt.

## Patentansprüche

1. Inulinester, die mindestens 6, vorzugsweise 6 bis 50 miteinander verbundene Fructoseeinheiten enthalten, wobei von den Hydroxylgruppen des Inulins mindestens eine mit einer gesättigten, 2 bis 22 Kohlenstoffatome aufweisenden Carbonsäure verestert ist, wobei der Substitutionsgrad (DS) < = 0,5 beträgt und wobei die Carbonsäure ausgewählt ist aus der Gruppe bestehend aus Essigsäure, Propionsäure, Buttersäure, Isobuttersäure, Valeriansäure, Capronsäure, Önanthsäure, Caprylsäure, Caprinsäure, Laurinsäure, Myristinsäure, Palmitinsäure, Stearinsäure, Arachinsäure und Behensäure.

2. Inulinester nach Anspruch 1, wobei die Carbonsäure 2 bis 7 Kohlenstoffatome aufweist.

3. Inulinester nach Anspruch 1 oder 2, wobei die Hydroxylgruppen mit unterschiedlichen Carbonsäuren verestert sind.

4. Inulinester nach Anspruch 1 oder 2, wobei die Hydroxylgruppen mit identischen Carbonsäuren verestert sind.

5. Wässrige Lösung enthaltend einen Inulinester nach einem der Ansprüche 1 bis 4.

6. Pulver, enthaltend einen Inulinester nach einem der Ansprüche 1 bis 4.

7. Kosmetikprodukt, insbesondere Reinigungscreme, Haarspülmittel und Körperlotion enthaltend einen Inulinester nach einem der Ansprüche 1 bis 4.

8. Spülmittel enthaltend einen Inulinester nach einem der Ansprüche 1 bis 4.

9. Verfahren zur Herstellung eines Inulinesters nach einem der Ansprüche 1 bis 4, **dadurch gekennzeichent,** daß mindestens 6, vorzugsweise 6 bis 50 miteinander verbundene Fructoseeinheiten aufweisendes Inulin mit Chloriden oder Anhydriden 2 bis 22, vorzugsweise 2 bis 7, Kohlenstoffatome aufweisender Carbonsäuren oder Gemischen davon umgesetzt wird, wobei der einzige Lösungsmittel Pyridin ist.

10. Verfahren zur Herstellung eines Inulinesters nach einem der Ansprüche 1 bis 4, **dadurch gekennzeichent,** daß mindestens 6, vorzugsweise 6 bis 50 miteinander verbundene Fructoseeinheiten aufweisendes Inulin mit Anhydriden 2 bis 22, vorzugsweise 2 bis 7, Kohlenstoffatome aufweisender Carbonsäuren oder Gemischen davon umgesetzt wird, wobei als einziges Lösungsmittel Wasser verwendet wird.

11. Verfahren zur Herstellung eines Inulinesters nach einem der Ansprüche 1 bis 4, **dadurch gekennzeichnet, daß** mindestens 6, vorzugsweise 6 bis 50 miteinander verbundene Fructoseeinheiten aufweisendes Inulin mit Anhydriden 2 bis 22, vorzugsweise 2 bis 7, Kohlenstoffatome aufweisender Carbonsäuren oder Gemischen davon lösungsmittelfrei umgesetzt wird.

12. Verfahren zur Herstellung eines Inulinesters nach einem der Ansprüche 1 bis 4, **dadurch gekennzeichnet, daß** mindestens 6, vorzugsweise 6 bis 50 miteinander verbundene Fructoseeinheiten aufweisendes Inulin mit Carbonsäureester 2 bis 22, vorzugsweise 12 bis 22, Kohlenstoffatome aufweisender Carbonsäuren oder Gemischen davon lösungsmittelfrei mit Katalysator umgesetzt wird.

13. Verfahren zur Herstellung eines Inulinesters nach Anspruch 12, **dadurch gekennzeichnet, daß** das Verfahren in einem Extruder oder Kneter durchgeführt wird.

14. Verfahren nach einem der Ansprüche 9 bis 11, wobei zusätzlich ein Katalysator verwendet wird.

15. Verfahren nach Anspruch 12, wobei basische oder saure Katalysatoren verwendet werden.

16. Verfahren nach Anspruch 13, wobei als Katalysatoren 4-(Dimethylamino)-pyridin, Natriumacetat, Kaliumcarbonat, Ionenaustauscher in der H⁺, OH-Form oder Pyridin verwendet werden.

17. Verwendung von Inulinestern nach einem der Ansprüche 1 bis 4 oder Gemischen davon als grenzflächenaktive Substanzen, als Additiv in Wasch-, Spülund Reinigungsmitteln, als Weichmacher oder als Hilfsstoff in der Papier-, Textil- und Lackindustrie.

## Claims

1. Inulin esters which contain at least 6, preferably 6 to 50 fructose units bonded to one another, wherein at least one of the hydroxyl groups of the inulin is esterified with a saturated carboxylic acid having 2 to 22 carbon atoms, wherein the degree of substitution (DS) amount to ≤ 0.5 and wherein the carboxylic acid is selected from the group consisting of acetic acid, propionic acid, butyric acid, isobutyric acid, valeric acid, capronic acid, oenanthic acid, caprylic acid, capric acid, lauric acid, myristic acid, palmitic acid, stearic acid, arachic acid and behemic acid.

2. An inulin ester according to claim 1, wherein the carboxylic acid has 2 to 7 carbon atoms.

3. An inulin ester according to claim 1 or 2, wherein the hydroxyl groups are esterified with different carboxylic acids.

4. An inulin ester according to claim 1 or 2, wherein the hydroxyl groups are esterified with identical carboxylic acids.

5. An aqueous solution containing an inulin ester according to any of claims 1 to 4.

6. A powder containing an inulin ester according to any of claims 1 to 4.

7. A cosmetic product, especially a cleansing cream, a hair rinse or a body lotion, containing an inulin ester according to any of claims 1 to 4.

8. A rinse containing an inulin ester according to any of claims 1 to 4.

9. A method of making an inulin ester according to any of claims 1 to 4, **characterized in that** an inulin having at least 6 and preferably 6 to 50 fructose units bonded to one another is reacted with chlorides or anhydrides of carboxylic acids having 2 to 22, preferably 2 to 7 carbon atoms or mixtures thereof, wherein pyridine is the sole solvent.

10. A method of making an inulin ester according to any of claims 1 to 4, **characterized in that** an inulin having at least 6 and preferably 6 to 50 fructose units bonded to one another is reacted with anhydrides of carboxylic acids having 2 to 22, preferably 2 to 7 carbon atoms or mixtures thereof, wherein water is used as the sole solvent.

11. A method of making an inulin ester according to any of claims 1 to 4, **characterized in that** an inulin having at least 6 and preferably 6 to 50 fructose units bonded to one another is reacted without a solvent with anhydrides of carboxylic acids having 2 to 22, preferably 2 to 7 carbon atoms or mixtures thereof.

12. A method of making an inulin ester according to any of claims 1 to 4, **characterized in that** an inulin having at least 6 and preferably 6 to 50 fructose units bonded to one another is reacted without a solvent but with a catalyst with carbonic esters of carboxylic acids having 2 to 22, preferably 12 to 22 carbon atoms or mixtures thereof.

13. A method of making and inulin ester according to claim 12, **characterized in that** the method is carried out in an extruder or a kneader.

14. A method according to any of claims 9 to 11, wherein a catalyst is additionally used.

15. A method according to claim 12, wherein basic or acidic catalysts are used.

16. A method according to claim 13, wherein 4-(dimethylamino)-pyridine, sodium acetate, potassium carbonate, ion exchangers in the H⁺, OH⁻ form or pyridine are used as catalysts.

17. Use of inulin esters according to any of claims 1 to 4 or mixtures thereof as surface-active agents, as additives in washing, rinsing and cleaning agents, as softeners or as auxiliary agents in the paper, textile and paint industries.

## Revendications

1. Esters d'inuline qui contiennent au moins 6, de préférence de 6 à 50 éléments de fructose reliés les uns aux autres, dans lesquels parmi les groupes hydroxyle de l'inuline au moins un est estérifié par un acide carboxylique saturé possédant de 2 à 22 atomes de carbone, le degré de substitution (DS) est ≤ 0,5, les acides carboxyliques étant choisis dans le groupe qui consiste en l'acide acétique, l'acide propionique, l'acide butyrique, l'acide isobutyrique, l'acide valérianique, l'acide caproïque, l'acide oenanthique, l'acide caprylique, l'acide caprique, l'acide laurique, l'acide myristique, l'acide palmitique, l'acide stéarique, l'acide arachique et l'acide béhénique.

2. Ester d'inuline selon la revendication 1,
dans lequel
l'acide carboxylique possède de 2 à 7 atomes de carbone.

3. Ester d'inuline selon la revendication 1 ou la revendication 2,
dans lequel
les groupes hydroxyle sont estérifiés avec des acides carboxyliques différents.

4. Ester d'inuline selon la revendication 1 ou la revendication 2,
dans lequel
les groupes hydroxyle sont estérifiés avec des acides carboxyliques identiques.

5. Solution aqueuse contenant un ester d'inuline selon l'une quelconque des revendications 1 à 4.

6. Poudre contenant un ester d'inuline selon l'une quelconque des revendication 1 à 4.

7. Produit cosmétique, en particulier crème de nettoyage, produit de lavage des cheveux et lotion corporelle, contenant un ester d'inuline selon l'une quelconque des revendications 1 à 4.

8. Agent de lavage contenant un ester d'inuline selon l'une quelconque des revendications 1 à 4.

9. Procédé de préparation d'un ester d'inuline selon l'une quelconque des revendications 1 à 4,
**caractérisé en ce que**
de l'inuline possédant au moins 6, de préférence de 6 à 50 éléments de fructose reliés les uns aux autres, est mise à réagir avec des chlorures ou des anhydrides d'acide carboxylique possédant de 2 à 22, de préférence de 2 à 7 atomes de carbone ou des mélanges de ceux-ci, la pyridine étant le solvant unique.

10. Procédé de préparation d'un ester d'inuline selon l'une quelconque des revendications 1 à 4,
**caractérisé en ce que**
de l'inuline possédant au moins 6, de préférence de 6 à 50 éléments de fructose reliés les uns aux autres, est mise à réagir avec des anhydrides d'acide carboxylique possédant de 2 à 22, de préférence de 2 à 7 atomes de carbone ou des mélanges de ceux-ci, avec comme solvant unique, de l'eau.

11. Procédé de préparation d'un ester d'inuline selon l'une quelconque des revendications 1 à 4,
**caractérisé en ce que**
de l'inuline possédant au moins 6, de préférence de 6 à 50 éléments de fructose reliés les uns aux autres, est mise à réagir avec des anhydrides d'acide carboxylique possédant de 2 à 22, de préférence de 2 à 7 atomes de carbone, ou des mélanges de ceux-ci, sans solvant.

12. Procédé de préparation d'un ester d'inuline selon l'une quelconque des revendications 1 à 4,
**caractérisé en ce que**
de l'inuline possédant au moins 6, de préférence de 6 à 50 éléments de fructose reliés les uns aux autres est mise à réagir avec un ester d'acide carboxylique d'acide carboxylique ayant de 2 à 22 de préférence de 12 à 22 atomes de carbone ou des mélanges de ceux-ci, sans solvant, avec un catalyseur.

13. Procédé de préparation d'un ester d'inuline selon la revendication 12,
**caractérisé en ce que**
le procédé est exécuté dans une extrudeuse ou dans un malaxeur.

14. Procédé selon l'une quelconque des revendications 9 à 11,
dans lequel,
on utilise en supplément un catalyseur.

15. Procédé selon la revendication 12, dans lequel on utilise des catalyseurs basiques ou acides.

16. Procédé selon la revendication 13, dans lequel comme catalyseurs on utilise la 4-diméthylamino)pyridine, l'acétate de sodium, le carbonate de potassium, un échangeur d'ions sous la forme H⁺ ou sous la forme OH⁻ ou bien la pyridine.

17. Utilisation des esters d'inuline selon l'une quelconque des revendications 1 à 4, ou de mélanges de ceux-ci en tant que substances actives sur la tension superficielle, comme additifs dans des produits de lavage, de rinçage et de nettoyage, comme agent plastifiant ou comme adjuvant dans l'industrie du papier, du textile et des laques.
